# EUROPEAN PATENT SPECIFICATION

(11) **EP 2 986 157 B1**
(45) Date of publication and mention of the grant of the patent: **12.02.2020**
(21) Application number: 14717516.0
(22) Date of filing: 08.04.2014
(51) Int. Cl.: A23L 33/10, A23L 33/00, A23C 9/123, A61K 35/74, A61P 1/12

(54) **FERMENTED NUTRITIONAL COMPOSITION WITH THIOL PROTEASE INHIBITOR**
FERMENTIERTE NAHRUNGSZUSAMMENSETZUNG MIT THIOLPROTEASEINHIBITOREN
COMPOSITION NUTRITIONNELLE FERMENTÉE AVEC INHIBITEUR DE PROTÉASES DE THIOL

(30) Priority: 08.04.2013 EP 13001784
(43) Date of publication of application: 24.02.2016
(73) Proprietor: N.V. Nutricia, 2712 HM Zoetermeer (NL)
(72) Inventor: RENES, Ingrid Brunhilde, NL-3584 CR Utrecht (NL); LUDWIG, Thomas, NL-3584 CT Utrecht (NL); BOURITIUS, Houkje, NL-3584 CT Utrecht (NL); LAMBERT, Johanna Maria, NL-3584 CT Utrecht (NL); HUYBERS, Sylvie, 5707 EM Helmond (NL); ABRAHAMSE, Evan, NL-3584 CT Utrecht (NL)
(74) Representative: Nederlandsch Octrooibureau
(86) International application number: PCT/NL2014/050221
(87) International publication number: WO 2014/168474

(56) References cited:
- WO-A1-2011/092261
- WO-A1-2012/078039
- WO-A1-2013/187755
- JP-A- 2011 172 506
- F. TURRONI ET AL: "Characterization of the Serpin-Encoding Gene of Bifidobacterium breve 210B", APPLIED AND ENVIRONMENTAL MICROBIOLOGY, vol. 76, no. 10, 26 March 2010 (2010-03-26), pages 3206-3219, XP055074606, ISSN: 0099-2240, DOI: 10.1128/AEM.02938-09 cited in the application
- JEAN PAUL MOTTA ET AL: "Food-grade Lactic Acid bacteria Expressing Elastatse Inhibitors Protect from Intestinal Inflammation in acute and chronic models of Colitis in Mice", GASTROENTEROLOGY, vol. 142, no. 5, S1, May 2012 (2012-05), page S720, XP055074636, ISSN: 0016-5085
- AIT-BELGNAOUI A ET AL: "T1454 Intracolonic Infusion of Fecal Supernatants from Diarrhea-Predominant Irritable Bowel Syndrome Patients (IBS-D) Evokes Visceral Hyperalgesia in Mice: Reversal By a Probiotic Treatment Via a Decrease of Luminal Serine-Protease Activity", GASTROENTEROLOGY, ELSEVIER, PHILADELPHIA, PA, vol. 134, no. 4, 1 April 2008 (2008-04-01) , pages A-559, XP023434405, ISSN: 0016-5085 [retrieved on 2008-04-01]
- AGRAWAL A ET AL: "T1395 Fermented Milk Containing the Probiotic Bifidobacterium Animalis, DN-173 010 (FM) Improves Abdominal Distension, Bloating and Transit in Irritable Bowel Syndrome with Constipation (IBS-C)", GASTROENTEROLOGY, ELSEVIER, PHILADELPHIA, PA, vol. 134, no. 4, 1 April 2008 (2008-04-01) , pages A-546, XP023434346, ISSN: 0016-5085, DOI: 10.1016/S0016-5085(08)62549-4 [retrieved on 2008-04-01]
- THIBAULT H ET AL: "EFFECTS OF LONG-TERM CONSUMPTION OF A FERMENTED INFANT FORMULA (WITH BIFIDOBACTERIUM BREVE C50 AND STREPTOCOCCUS THERMOPHILUS 065) ON ACUTE DIARRHEA IN HEALTHY INFANTS", JOURNAL OF PEDIATRIC GASTROENTEROLOGY AND NUTRITION, LIPPINCOTT WILLIAMS WILKINS, INC, US, vol. 39, no. 2, 1 August 2004 (2004-08-01) , pages 147-152, XP009043383, ISSN: 0277-2116, DOI: 10.1097/00005176-200408000-00004

## Description

### FIELD OF THE INVENTION

The present field relates to nutritional compositions comprising protein and thiol protease inhibitor activity.

### BACKGROUND OF THE INVENTION

Thiol proteases, also named cysteine proteases, are characterized in that they have a thiol group in their active center. Typically these proteases are not part of the proteases of the mammalian digestive system, in contrast to serine proteases. Known thiol proteases are cathepsin (L, B, K), calcium-dependent neutral protease (CAMP), papain, and bromelain.

In the gastro-intestinal tract such activity is typically displayed by cathepsins expressed by macrophages and epithelial cells and by bacterial or viral thiol proteases. A too high intestinal thiol protease activity is associated with tissue damage and gastro-intestinal inflammation and plays a role in infection.

Human milk contains a variety of protease inhibitors, such as lactoferrin, β-casein, cystatins, α1-anti-trypsin, and α1-anti-chymotrypsin. Protease inhibitors limit digestive and bacteria-induced protease activity to prevent protease-induced tissue injury. Inhibition of thiol protease activity with milk ingredients are known in the art. EP 1568377 discloses the use of casein or casein hydrolysates as cysteine protease inhibitor. EP 1576964 discloses the use of lactoferrin or lactoferrin hydrolysates as cysteine protease inhibitor. WO 2012/078038 discloses that a fermented infant formula reduces the amount of digestive protease activity in the intestinal tract. Turroni et al., 2010, AEM 76:3206-3219 discloses the characterization of a serine protease inhibitor gene in a *Bifidobacterium breve* strain.

### SUMMARY OF THE INVENTION

The inventors found that the inhibition capacity of the thiol protease activity of milk-derived nutritional compositions was increased after a fermentation step by lactic acid bacteria, when compared to non-fermented milk-derived nutritional compositions similar in macro-and micronutrient composition and under conditions of similar pH.

In particular fermentation by *Streptococcus* contributed to this effect. As the fermentation by *Streptoccoccus thermophilus* did not result in hydrolysis of larger proteins, this effect cannot be attributed to the hydrolysis of the casein or lactoferrin present in the milk protein.

Papain was used as a model thiol protease. Since thiol proteases are not very important for protein digestion, but play an important role in inflammation, consumption of a fermented milk-derived nutritional consumption may beneficially decrease thiol protease activity in the gastro-intestinal tract, thereby contributing to the gastro-intestinal health. Reduction of thiol protease activity in the gastrointestinal tract will result in treatment or prevention of mild gastrointestinal inflammation, IBD, IBS and diminish colics, intestinal cramps and abdominal pain and gastrointestinal infections.

This is of particular importance for infants and young children as their gastrointestinal tract is still developing and they have a developing microbiota, often concomitant with a state of mild gastro-inflammation, a decreased gastro-intestinal barrier function, and intestinal discomfort.

### DETAILED DESCRIPTION OF THE INVENTION

The present invention, which is defined by the claims, relates to a method for inhibiting thiol protease activity, comprising contacting thiol protease with a composition comprising a milk-derived fermented product that is fermented by lactic acid producing bacteria, the milk-derived fermented product comprising i) lactic acid and/or lactate, ii) at least one selected from the group consisting of whey, whey protein and casein.

In other words the invention relates to the use of a composition comprising a milk-derived fermented product that is fermented by lactic acid producing bacteria, the milk-derived fermented product comprising i) lactic acid and/or lactate, ii) at least one selected from the group consisting of whey, whey protein and casein, for inhibiting thiol protease activity.

The invention also relates to the use of a milk-derived fermented product that is fermented by lactic acid producing bacteria, the fermented milk-derived product comprising i) lactic acid and/or lactate, ii) at least one selected from the group consisting of whey, whey protein and casein, in the manufacture of a nutritional composition for treatment and/or prevention of thiol protease mediated disorders selected from the group consisting of gastro-intestinal inflammation, inflammatory bowel disease, irritable bowel syndrome, colics, intestinal cramps, abdominal pain and gastrointestinal infections, by reducing thiol protease activity, preferably by reducing thiol protease activity in the gastrointestinal tract.

The invention in a first aspect concerns a nutritional composition comprising a milk-derived fermented product that is fermented by lactic acid producing bacteria, the milk-derived fermented product comprising i) lactic acid and/or lactate, ii) at least one selected from the group consisting of whey, whey protein and casein for use in the treatment and/or prevention of thiol protease mediated gastro-intestinal inflammation, by reducing thiol protease inhibitor activity, preferably by reducing thiol protease activity in the gastrointestinal tract, wherein said milk-derived product is fermented with S. *thermophilus.*

In a second aspect, the invention concerns the use of fermentation by *S*. *thermophilus* of a nutritional composition that comprises milk or comprises lactose and at least one selected from the group consisting of whey, whey protein, and casein, for increasing the thiol protease activity of a nutritional composition.

It is noted that wherever in the present description wording like "the present nutritional composition" or "nutritional composition according to the (present) invention" is used, this also refers to the methods and uses according to the present invention.

### Thiol protease inhibitor

The term 'protease' as used herein refers to enzymes which are capable of hydrolyzing proteins and/or peptides by cleavage of the peptide bond. The term 'thiol protease' as used herein refers to proteases with a thiol group in the active center. The active thiol groups of these enzymes are derived from the cysteine residues present. The term thiol protease inhibitor as used in connection with the present invention refers to a compound, a substance and/or composition which is capable of inhibiting the action of the thiol protease. More preferably the thiol protease activity that is inhibited is that of papain and cathepsins, such as cathepsin B, D, and L.

Thiol protease activity, such as papain, can be measured as known in the art (see also the examples). One unit of papain activity is defined as the hydrolysis of 1.0 µmole of N-α-benzoyl-L-arginine ethyl ester (BAEE) per minute at pH 6.2 at 25 °C (according to (product information sheet of supplier Sigma-Aldrich). Papain is also known as papainase or papaya peptidase I (EC 3.4.22.2).

One unit of thiol protease inhibitor (TIU) is defined as the amount of inhibitor that inhibits 50% of the thiol protease preparation, which has an activity of 2 units. One unit of papain inhibitor (PIU) is defined as the amount of inhibitor that inhibits 50% of the papain preparation, which has an activity of 2 units. In one embodiment of the method or use according to the present invention, the the nutritional composition has a thiol protease inhibitor activity of at least 40 papain inhibitor units (PIU) per g dry weight of the nutritional composition.

### Fermented product

Fermentation is the process of deriving energy from the oxidation of carbohydrates, such as the lactose present in milk, using an endogenous electron acceptor, which is usually an organic compound. This is in contrast to cellular respiration, where electrons are donated to an exogenous electron acceptor, such as oxygen, via an electron transport chain. In the present invention fermentation of a milk-derived product by lactic acid producing bacteria has the common meaning of the conversion of carbohydrates present in the milk-derived product to organic acids. These organic acids formed may comprise, besides lactic acid, also other organic acids such as acetate. Lactic acid bacteria are also referred to as lactic acid producing bacteria and include bacteria of the genus Streptococcus, Lactococcus, Lactobacillus, Leuconostoc, Enterococcus, Oenococcus, Pediococcus, and Bifidobacterium. Preferably the milk-derived product is not fermented by *Lactobacillus bulgaricus. L. bulgaricus* fermented products are considered not suitable for infants, since in infants the specific dehydrogenase that converts D-lactate to pyruvate is far less active than the dehydrogenase which converts L-lactate.

According to the present invention, the nutritional composition comprises a milk-derived product that is fermented with *S*. *thermophilus* as lactic acid producing bacteria. The fermented milk-derived product comprises lactic acid and/or lactate. The fermented milk-derived product further comprises one or more selected from the group consisting of whey, whey protein, whey protein hydrolysate, casein and casein hydrolysate. Optionally the fermented milk-derived product may comprises one or more selected from the group consisting of whey protein hydrolysate and casein hydrolysate. The term 'fermented milk-derived product' includes fermented milk. The fermented milk-derived product is obtained by incubation of a combination of milk, e.g. skim milk, or by incubation of a combination of a composition comprising lactose and preferably one or more selected from the group consisting of whey, whey protein, whey protein hydrolysate, casein and casein hydrolysate, with at least one lactic acid producing bacterium, namely at least *Streptococcus thermophilus.* Preferably the combination is incubated for 10 minutes to about 6 hours. The temperature during incubation is preferably from 20 to 50 °C. After incubation the incubated product is preferably subjected to a heat treatment. By this heat treatment preferably at least 90 % of living lactic acid bacteria are inactivated. Thus in one embodiment according to the present invention the lactic acid producing bacteria in the nutritional composition are inactivated and/or non-replicating. The heat treatment preferably is performed at a temperature from 80 to 180 °C. Procedures to prepare fermented milk-derived products suitable for the purpose of the present invention are known per se. EP 778885, which is incorporated herein by reference, discloses in particular in example 7 a suitable process for preparing a fermented milk-derived product. FR 2723960, which is incorporated herein by reference, discloses in particular in example 6 a suitable process for preparing a fermented milk-derived product.

Briefly, a milk-derived product, preferably pasteurised, containing lactose and optionally further macronutrients such as (vegetable) fats, casein, whey protein, vitamins and/or minerals etc. is concentrated, e.g. to a value 15 of 50% dry matter and then inoculated with *S. thermophilus,* for example with 5% of a culture containing to 10¹⁰ bacteria per ml. Temperature and duration of fermentation are as mentioned above. Suitably after fermentation the fermented milk-derived product may be pasteurised or sterilised and for example spray dried or lyophilised to provide a form suitable to be formulated in the end product.

The bacterial strains of *S. thermophilus* that are preferably used to prepare the fermented milk-derived product for the purpose of the present invention develop beta-galactosidase activity in the course of fermentation of the substrate. Preferably beta-galactosidase activity develops in parallel with acidity. Preferably a beta-galactosidase activity develops which is sufficient to permit subsequent enrichment of the fermented milk-derived product in galactooligosaccharides. Thus preferably suitable *S. thermophilus* strains, when cultured on a medium containing lactose, in particular a medium based on milk concentrate, achieve fermentation of the medium accompanied by high production of galactooligosaccharides. Selection of a suitable strain of *S*. *thermophilus* is described in example 2 of EP 778885 and in example 1 of FR 2723960. Preferred strains of *S. thermophilus* to prepare the fermented milk-derived products for the purpose of the present invention have been deposited by Compagnie Gervais Danone at the Collection Nationale de Cultures de Microorganismes (CNCM) run by the Institut Pasteur, 25 rue du Docteur Roux, Paris, France on 23 August 1995 under the accession number 1-1620 and on 25 August 1994 under the accession number 1-1470. Preferably, in the preparation of the fermented milk-derived product additionally other strains of lactic acid bacteria are present or, either simultaneously or consecutively, the fermented milk-derived product additionally is fermented by other strains of lactic acid bacteria. Other strains of lactic acid bacteria are preferably selected from the group consisting of Lactobacillus and Bifidobacteria, more preferably *Bifidobacterium breve,* most preferably Bifidobacterium *B. breve* strain deposited by Compagnie Gervais Danone at the CNCM under number 1-2219 on 31 May, 1999. Preferably the milk-derived product is fermented by *Streptococcus thermophilus* and/or *Bifidobacterium breve.* Preferably the nutritional composition comprises *Streptococcus thermophilus* and/or *Bifidobacterium breve.* Preferably the lactic acid producing bacteria in the nutritional composition are present in inactivated and/or non-replicating form, as it prevents the further degradation of the high amounts of lactose present in the product.

The present composition preferably comprises at least 5 wt.% based on dry weight of the total product, of the fermented milk-derived product. Preferably the composition comprises at least 10 wt.%, more preferably at least 25 wt.%, even more preferably at least 40 wt.% based on dry weight of the total product, of the fermented milk-derived product. The present composition comprises at most 100 wt.% based on dry weight of the total product, of the fermented milk-derived product. Preferably the composition comprises at most 90 wt.%, more preferably at most 70 wt.%, even more preferably at most 50 wt.% based on dry weight of the total product, of the fermented milk-derived product.

The inventors found that specifically the thiol protease activity inhibitory capacity of milk-derived nutritional compositions was increased by fermentation by lactic acid bacteria, compared to non-fermented milk-derived nutritional compositions similar in macro- and micronutrient composition and under conditions of similar pH. In particular fermentation by *Streptococcus* contributed to this effect. According to the present invention, upon fermentation by *Streptoccoccus thermophilus* no protein degradation products were found with conventional analyses like gel electrophoresis or column chromatography, and as sufficient free amino acids were present to prevent the expression of proteases, the increased thiol protease activity inhibitory effect cannot be attributed to the hydrolysis of the casein or lactoferrin present in the milk protein. Without wishing to be bound by theory the inventors believe that incubation of the milk-derived components under fermentation conditions (thereby slowly decreasing the pH and/or the formation of lactic acid), the components are slightly modified and the naturally present capacity to inhibit thiol protease activity is enhanced. Alternatively, the effect of the presence of lactic acid and/or decreased pH on the components responsible for inhibition thiol protease activity is enhancing the capacity. It is noted that the papain activity and inhibition measurements are performed in an assay with constant pH.

Therefore, the present invention also relates to a milk-derived nutritional composition comprising 0.10 to 1.5 wt.% of the sum of lactic acid and lactate based on dry weight of the nutritional composition, wherein the sum of L-lactic acid and L-lactate is more than 50 wt.% based on the sum of total lactic acid and lactate and said nutritional composition having a pH of 5.0 to 6.5. More preferably the present milk-derived nutritional composition comprises with 0.20 to 1.0 wt.% of the sum of lactic acid. More preferably the sum of L-lactic acid and L-lactate is more than 90 wt.% based on the sum of total lactic acid and lactate. More preferably the milk-derived nutritional composition has a pH of 5.5 to 6.3.

The inhibition of digestive enzymes, mainly serine proteases, by fermented milk-derived nutritional compositions was observed to a lesser extent, which is beneficial, as too much inhibition of serine protease activity will interfere with normal protein digestion.

### Nutritional composition

The present nutritional composition is preferably particularly suitable for providing the complete daily nutritional requirements to an infant or a young child, or in other words to a human subject with an age of 0 to 36 months, more preferably to an infant, or in other words to a human subject with an age of 0 to 12 months. The present nutritional composition is preferably not a yogurt, since yoghurt contains by convention *L. bulgaricus* (Codex Standard for fermented Milks Codex Stan 243-2003).

The present nutritional composition comprises digestible carbohydrate, in particular lactose. Thus herein, lactose is considered to be a digestible carbohydrate. However, also other digestible carbohydrates such as glucose, sucrose, fructose, galactose, maltose, starch and maltodextrin may be present. Preferably the present nutritional composition does not comprise high amounts of digestible carbohydrates other than lactose. When in liquid form, e.g. as a ready-to-feed liquid, the nutritional composition preferably comprises 6.0 to 30 g digestible carbohydrate per 100 ml, more preferably 6.0 to 20, even more preferably 7.0 to 10.0 g per 100 ml. Based on dry weight the present nutritional composition preferably comprises 40 to 80 wt.%, more preferably 40 to 65 wt.% digestible carbohydrates. Based on total calories the nutritional composition comprises 9 to 20 g digestible carbohydrates per 100 kcal, more preferably 9 to 15 g.

The present nutritional composition preferably comprises lipid. The lipid of the present nutritional composition provides 3 to 7 g per 100 kcal of the nutritional composition, preferably the lipid provides 4 to 6 g per 100 kcal. When in liquid form, e.g. as a ready-to-feed liquid, the nutritional composition preferably comprises 2.1 to 6.5 g lipid per 100 ml, more preferably 3.0 to 4.0 g per 100 ml. Based on dry weight the present nutritional composition preferably comprises 12.5 to 40 wt.% lipid, more preferably 19 to 30 wt.%. Preferably the lipid comprises the essential fatty acids alpha-linolenic acid (ALA), linoleic acid (LA) and/or long chain polyunsaturated fatty acids (LC-PUFA). The LC-PUFA, LA and/or ALA may be provided as free fatty acids, in triglyceride form, in diglyceride form, in monoglyceride form, in phospholipid form, or as a mixture of one of more of the above. Preferably the present nutritional composition contains at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil and milk fat.

Preferably the present nutritional composition comprises protein. The protein is preferably selected from the group consisting of non-human animal proteins, preferably milk proteins. Preferably the present nutritional composition comprises one or more selected from the group consisting of whey, whey protein, whey protein hydrolysate, casein and casein hydrolysate. The nutritional composition preferably contains casein, and/or whey protein, more preferably bovine whey proteins and/or bovine casein. The nutritional composition preferably comprises casein and whey proteins in a weight ratio casein:whey protein of 10:90 to 90:10, more preferably 20:80 to 80:20, even more preferably 35:65 to 55:45.

The nutritional composition of the present invention preferably provides protein in an amount of 1.25 to 4 g per 100 kcal, preferably providing 1.5 to 3 g, even more preferable 1.7 to 2.5 g per 100 kcal. When in liquid form, the nutritional composition preferably comprises 0.5 to 6.0 g, more preferably 1.0 to 3.0 g, even more preferably 1.0 to 1.5 g protein per 100 ml, most preferably 1.0 to 1.3 g protein per 100 ml. Based on dry weight the present nutritional composition preferably comprises 5 to 20 wt.% protein, preferably at least 8 wt.%, more preferably 8 to 14 wt.%, protein even more preferably 8 to 9.5 wt.% based on dry weight of the nutritional composition.
In one embodiment, the nutritional composition preferably comprises casein and whey proteins in a weight ratio casein:whey protein of 10:90 to 90:10, more preferably 20:80 to 80:20, even more preferably 35:65 to 55:45, and comprises 5 to 20 wt.% protein, preferably at least 8 wt.%, more preferably 8 to 14 wt.%, protein even more preferably 8 to 9.5 wt.% based on dry weight of the nutritional composition.

The nutritional composition of the present invention preferably provides lipid in an amount of 3 to 7 g per 100 kcal, preferably 4 to 6 g per 100 kcal, protein in an amount of 1,25 to 4 g per 100 kcal, preferably 1.5 or 1.6 to 3 g per 100 kcal, preferably 1.7 to 2.5 g per 100 kcal and digestible carbohydrate in an amount of 5 to 20 g per 100 kcal, preferably 8 to 15 g per 100 kcal of the nutritional composition. Preferably the present nutritional composition comprises lipid providing 4 to 6 g per 100 kcal, protein providing 1.6 to 1.9 g per 100 kcal, more preferably 1.75 to 1.85 g per 100 kcal and digestible carbohydrate providing 8 to 15 g per 100 kcal of the final nutritional composition.

The amount of total calories is determined by the sum of calories derived from protein, lipids, digestible carbohydrates and non-digestible oligosaccharides. Protein and carbohydrates are considered to have a caloric density of 4 kcal/g, fat of 9 kcal/g and non-digestible oligosaccharides 2 kcal/g.

The present nutritional composition is not human breast milk. The nutritional composition according to the invention or the nutritional composition used according to the invention preferably comprises other fractions, such as vitamins, minerals, trace elements and other micronutrients in order to make it a complete nutritional composition. Preferably the nutritional composition is selected from the group consisting of an infant formula, follow on formula, toddler milk or formula and growing up milk, more preferably form the group consisting of an infant formula. An infant formula is defined as a formula for use in infants and can for example be a starter formula, intended for infants of 0 to 4 to 6 months of age or a follow on formula, intended for infants of 4 to 6 months until 12 months of age. A toddler milk or growing up milk or formula is intended for children of 12 to 36 months of age. In one embodiment the nutritional composition is an infant formula. Infant formulae comprise vitamins, minerals, trace elements and other micronutrients according to international directives.

In one embodiment the nutritional composition is in a liquid form. In another embodiment the nutritional composition is a powder suitable for making a liquid nutritional composition after reconstitution with an aqueous solution, preferably with water. Preferably the nutritional composition is a powder, suitable for reconstitution with water to a liquid. Preferably the infant or toddler formula is a powder to be reconstituted with water. Preferably the liquid composition has a viscosity below 100 mPa.s, more preferably below 60 mPa.s, more preferably below 35 mPa.s, even more preferably below 6 mPa.s as measured in a Brookfield viscometer at 20°C at a shear rate of 100 s⁻¹. A low viscosity is important for infant or follow on formula, since it mimics the viscosity of breast milk and can then be administered via a teat.

The pH of the present nutritional composition when in ready to drink liquid form is preferably from 5.0 to 7.5, more preferably from 5.0 to 6.5, most preferably from 5.5 to 6.3.
In one embodiment, the nutritional composition according to the present invention is a liquid with a pH of 5.0-6.5 or a powder reconstitutable with water to a liquid with a pH of 5.0-6.5.

In order to meet the caloric requirements of an infant or toddler, the nutritional composition preferably comprises 45 to 200 kcal/100 ml liquid. For infants the nutritional composition has more preferably 60 to 90 kcal/100 ml liquid, even more preferably 65 to 75 kcal/100 ml liquid. This caloric density ensures an optimal ratio between water and calorie consumption. For toddlers, human subjects with an age from 12 to 36 months, the nutritional composition more preferably has a caloric density from 45 to 65, even more preferably from 50 to 60 kcal/100 ml. The osmolarity of the present composition is preferably from 150 to 420 mOsmol/l, more preferably from 260 to 320 mOsmol/l. The low osmolarity aims to further reduce the gastrointestinal stress.

When the nutritional composition is in a liquid form, the preferred volume administered on a daily basis is in the range of about 80 to 2500 ml, more preferably about 200 to 1200 ml per day. Preferably, the number of feedings per day is from 1 to 10, preferably from 3 to 8. In one embodiment the nutritional composition is administered daily for a period of at least 2 days, preferably for a period of at least 4 weeks, preferably for a period of at least 8 weeks, more preferably for a period of at least 12 weeks, in a liquid form wherein the total volume administered daily is from 200 ml to 1200 ml and wherein the number of feedings per day is from 1 to 10.

### Application

Preferably the present nutritional composition is suitable for, or suitable for administration to, a human subject. In one embodiment, the present nutritional composition is suitable for infants and/or young children. In one embodiment the present nutritional composition is for use in providing nutrition to human subjects with an age of 0 to 36 months. Young children, or toddlers, are defined as human subjects with an age of 12 to 36 months. Infants are defined as human subjects with an age of below 12 months. So in other words, the present nutritional composition is suitable for human subjects with an age of 0 to 36 months. Wherever in this description the term "infants and/or young children" is used, this can be replaced by "human subjects with an age of 0 to 36 months".

The present nutritional composition is preferably enterally administered, more preferably orally.

The present nutritional composition is preferably used for treatment and/or prevention mild gastrointestinal inflammation, IBD, IBS and diminish colics, intestinal cramps and abdominal pain and gastrointestinal infections by reduction of thiol protease activity, preferably in the gastrointestinal tract.

The present nutritional composition is preferably used for treatment and/or prevention of thiol protease mediated disorders selected from the group consisting of gastro-intestinal inflammation, inflammatory bowel disease, irritable bowel syndrome, colics, intestinal cramps, abdominal pain and gastrointestinal infections, by reducing thiol protease activity, preferably in the gastrointestinal tract, more preferably for treatment and/or prevention of gastro-intestinal inflammation, even more preferably mild intestinal inflammation, by reducing thiol protease activity in the gastrointestinal tract.

### EXAMPLES

### Example 1: fermented infant milk formula with papain inhibitory effect

A concentrated milk-derived product was prepared comprising preparing an aqueous phase with skim milk, whey protein, lactose, micro-ingredients, and a fat phase with vegetable fat, in amounts to make it suitable for an infant formula. The two phases were mixed and homogenized. The total solid content was about 41 wt.%. This product was kept at about 45 °C and inoculated with precultured *S. thermophilus* 065 and *B. breve* C50, both with about the same amount of cfu. The inoculum pregrowth and growth medium used was prepared from skim milk and 1 wt% yeast extract. Fermentation took place until the pH had dropped to about 5.2. The amount of lactic acid and lactate was then about 1 wt.% based on dry weight of the composition.
As a control the same milk-derived product with total solid of 41 wt.% was used, but which was not inoculated with the two lactic acid producing bacteria. The pH was neutral and no lactic acid and lactate were present.
Both products were cooled and spray dried to a powder. The powdered formula were reconstituted with water just before the assay at a concentration of 1.07 and 1.05 g/ml respectively, and had a pH of about 5.4-5.8.

Thiol Protease inhibition capacity was determined fluorimetrically based on Ivanov et al., 2006, JBC 281: 17246-17252. Papain (from Papaya latex, Sigma Aldrich P4762, 10 U per mg protein, 96% protein powder) was used as a reference. All reactions were performed in 50 mM sodium phosphate, pH 7.2, 100 mM NaCl buffer. The stock solutions of fluorogenic peptide substrate (N-Suc-AAPF-AMC) were prepared in Me₂SO and stored at -20 °C. The Me₂SO substrate stocks were diluted 75-fold into reaction buffer to prepare 2 X substrate buffers (400 µM MeOSuc-AAPF-AMC). Papain (5 U/ml) was then incubated for 30 min with various dilutions of formula samples (undiluted, 1:1, and 1:10 diluted). The enzymatic activity was subsequently assayed by rapidly mixing 50 µl of the substrate buffer with 50 µl of the enzyme/formula solution in a 96-well white plates and recording the fluorescence change on a FLUOstar (BMG Lab Tech, Ortenberg, Germany). Fluorescein intensity was measured from t=0 to t=20 minutes at 360-nm excitation and 460-nm emission wave lengths. The Vmax of each sample and standard was determined.

Digestive protease inhibition capacity was determined fluorimetrically with a standard assay kit (EnzCheck) for casein hydrolytic activity according to the protocol of the manufacturer and porcine pancreatin (Sigma P 175, 4^{∗}USP) was used as a reference. Briefly, samples were diluted in 10 mM Tris pH 7.8. Per well 50 µl undiluted, 1:2, 1:4, and 1:10 diluted sample and 50 µl pancreatin and was mixed and incubated for 30 min at 37 °C. Subsequently, 100 µl fluorescein labeled casein-substrate was added per well and fluorescein intensity was measured from t=0 to t=20 minutes at 485-nm excitation and 510-nm emission wavelengths using FLUOstar. Remaining protease activity of each sample was expressed as percentage of Vmax of the reference.
Table 1 shows that upon fermentation papain activity was reduced from 16.37 to 3.40, indicating that inhibitory activity was increased by a factor 4.8 by the fermentation.

**Table 1: Protease activity inhibition by a fermented or non fermented milk-derived nutritional composition.**

| Formula tested | % activity Vmax | | Inhibitory unit per g dry formula |
|---|---|---|---|
| | Papain (5U/ml added) in formula diluted 10 times | Pancreatin (0.1 mg/ml) in formula diluted 2 times | Papain |
| Ferment | 60.2^{∗} ± 6.1 | 29.2^{∗} ± 0.9 | 59.7 |
| Control, not fermented | 82.5 ± 4.9 | 45.9 ± 3.7 | 28.7 |
| No milk- product | 100 | 100 | 0 |

| | | | |
|---|---|---|---|
| * p<0.05 compared to control | | | |

### Example 2 Streptococcus thermophilus fermented milk with papain inhibitory effect.

A growth medium was prepared using skim milk and 1 wt% yeast extract. This medium was unfermented (control), or fermented with *S. thermophilus* 065 under conditions similar as in example 1, also to a final pH of about 5.2. Next, the papain activity was analyzed using the protocol described in example 1. Papain (5 U/ml and 2.5 U/ml) was then incubated for 30 min with various dilutions of formula samples (undiluted, 1:1, and 1:5 diluted). The enzymatic activity was subsequently assayed by rapidly mixing 50 µl of the substrate buffer with 50 µl of the enzyme/formula solution in a 96-well white plates and recording the fluorescence change on a FLUOstar (BMG Lab Tech, Ortenberg, Germany).

The results are shown in table 2. From Table 2 it can be deduced that fermentation of milk-derived product by Streptococcus alone already resulted in an increase in papain inhibition.

**Table 2: Papain activity inhibition by skim milk or skim milk fermented by Streptococcus thermophilus**

| Composition tested | % papain activity (% of 5 U/ml added) | % papain activity (% of 2.5 U/ml |
|---|---|---|
| Skim milk | 60.4% | 72.6% |
| Fermented skim milk | 38.7% | 52.9% |
| No milk | 100% | 100 |

## Claims

1. A nutritional composition comprising a milk-derived fermented product that is fermented by lactic acid producing bacteria, the milk-derived fermented product comprising i) lactic acid and/or lactate, ii) at least one selected from the group consisting of whey, whey protein and casein, for use in the treatment and/or prevention of gastro-intestinal inflammation mediated by thiol protease activity by reducing thiol protease activity, wherein said milk-derived product is fermented with *Streptococcus thermophilus* (*S. thermophilus*).

2. The nutritional composition for use according to claim 1, wherein the nutritional composition has a thiol protease inhibitor activity of at least 40 papain inhibitor units (PIU) based on g dry weight.

3. The nutritional composition for use according to claim 1 or 2, wherein the nutritional composition comprises 0.10 to 1.5 wt.% of the sum of lactic acid and lactate based on dry weight of the nutritional composition, wherein the sum of L-lactic acid and L-lactate is more than 50 wt.% based on the sum of total lactic acid and lactate.

4. The nutritional composition for use according to claim 1-3, wherein the lactic acid producing bacteria in the nutritional composition are inactivated and/or non-replicating.

5. The nutritional composition for use according to claim 1-4, wherein milk-derived product comprises casein and whey proteins in a weight ratio of 20:80 to 80:20, and comprises at least 8 wt.% protein based on dry weight of the nutritional composition.

6. The nutritional composition for use according to claim 1-5, wherein the thiol protease inhibitor is selected from papain inhibitor and cathepsin inhibitor.

7. The nutritional composition for use according to claim 1-7, wherein the nutritional composition is an infant formula, a follow on formula, or a growing up milk.

8. The nutritional composition for use according to claim 1-8, wherein said use comprises administering said nutritional composition to a to human subject with an age of 0 to 36 months.

9. Use of fermentation by *S. thermophilus* of a nutritional composition that comprises milk or comprises lactose and at least one selected from the group consisting of whey, whey protein and casein, for increasing the thiol protease activity of a nutritional composition.

10. The use according to claim 9, wherein the nutritional composition comprises 0.10 to 1.5 wt.% of the sum of lactic acid and lactate based on dry weight of the nutritional composition, wherein the sum of L-lactic acid and L-lactate is more than 50 wt.% based on the sum of total lactic acid and lactate.

11. The use according to claim 9-10, wherein the lactic acid producing bacteria in the nutritional composition are inactivated and/or non-replicating.

12. The use according to claim 9-11, wherein the nutritional composition comprises casein and whey proteins in a weight ratio of 20:80 to 80:20, and comprises at least 8 wt.% protein based on dry weight of the nutritional composition.

13. The use according to claim 9-12, wherein the thiol protease inhibitor is selected from papain inhibitor and cathepsin inhibitor.

14. The use according to claim 9-13, wherein the nutritional composition is an infant formula, a follow on formula, or a growing up milk.

## Patentansprüche

1. Nährstoffzusammensetzung, umfassend ein aus Milch gewonnenes, fermentiertes Produkt, das mit Hilfe von Milchsäure produzierenden Bakterien fermentiert wird, wobei das aus Milch gewonnene, fermentierte Produkt i) Milchsäure und/oder Lactat, und ii) mindestens eines gewählt aus der Gruppe, bestehend aus Molke, Molkenprotein und Casein, umfasst, zur Verwendung bei der Behandlung und/oder Vorbeugung einer Magen-Darm-Entzündung, die durch eine Thiolprotease-Aktivität herbeigeführt wird, durch Verminderung der Thiolprotease-Aktivität, wobei das aus Milch gewonnene Produkt mit *Streptococcus thermophilus* (*S. thermophilus*) fermentiert wird.

2. Nährstoffzusammensetzung zur Verwendung nach Anspruch 1, wobei die Nährstoffzusammensetzung eine Thiolprotease-Inhibitor-Aktivität von mindestens 40 Papain-Inhibitor-Einheiten (PIU), bezogen auf das Trockengewicht in Gramm, aufweist.

3. Nährstoffzusammensetzung zur Verwendung nach Anspruch 1 oder 2, wobei die Nährstoffzusammensetzung in der Summe 0,10 bis 1,5 Gew.-% Milchsäure und Lactat, bezogen auf das Trockengewicht der Nährstoffzusammensetzung, umfasst, wobei die Summe von L-Milchsäure und L-Lactat nicht mehr als 50 Gew.-%, bezogen auf die Summe von Milchsäure und Lactat insgesamt, beträgt.

4. Nährstoffzusammensetzung zur Verwendung nach Anspruch 1 bis 3, wobei die Milchsäure produzierenden Bakterien in der Nährstoffzusammensetzung inaktiviert und/oder nicht replizierend sind.

5. Nährstoffzusammensetzung zur Verwendung nach Anspruch 1 bis 4, wobei das aus Milch gewonnene Produkt Casein und Molkenproteine in einem Gewichtsverhältnis von 20:80 bis 80:20 umfasst und weiterhin mindestens 8 Gew.-% Protein, bezogen auf das Trockengewicht der Nährstoffzusammensetzung, umfasst.

6. Nährstoffzusammensetzung zur Verwendung nach Anspruch 1 bis 5, wobei der Thiolprotease-Inhibitor aus einem Papain-Inhibitor und einem Cathepsin-Inhibitor gewählt ist.

7. Nährstoffzusammensetzung zur Verwendung nach Anspruch 1 bis 6, wobei die Nährstoffzusammensetzung eine Säuglingsanfangsnahrung, eine Folgenahrung oder eine Wachstumsmilch ist.

8. Nährstoffzusammensetzung zur Verwendung nach Anspruch 1 bis 7, wobei die Verwendung das Verabreichen der Nährstoffzusammensetzung an einen Menschen im Alter von 0 bis 36 Monaten umfasst.

9. Verwendung einer Fermentation mit Hilfe von *S. thermophilus* bei einer Nährstoffzusammensetzung, die Milch umfasst oder die Lactose und mindestens eines gewählt aus der Gruppe, bestehend aus Molke, Molkenprotein und Casein, umfasst, zur Erhöhung der Thiolprotease-Aktivität einer Nährstoffzusammensetzung.

10. Verwendung nach Anspruch 9, wobei die Nährstoffzusammensetzung in der Summe 0,10 bis 1,5 Gew.-% Milchsäure und Lactat, bezogen auf das Trockengewicht der Nährstoffzusammensetzung, umfasst, wobei die Summe von L-Milchsäure und L-Lactat nicht mehr als 50 Gew.-%, bezogen auf die Summe von Milchsäure und Lactat insgesamt, beträgt.

11. Verwendung nach Anspruch 9 bis 10, wobei die Milchsäure produzierenden Bakterien in der Nährstoffzusammensetzung inaktiviert und/oder nicht replizierend sind.

12. Verwendung nach Anspruch 9 bis 11, wobei die Nährstoffzusammensetzung Casein und Molkenproteine in einem Gewichtsverhältnis von 20:80 bis 80:20 umfasst und weiterhin mindestens 8 Gew.-% Protein, bezogen auf das Trockengewicht der Nährstoffzusammensetzung, umfasst.

13. Verwendung nach Anspruch 9 bis 12, wobei der Thiolprotease-Inhibitor aus einem Papain-Inhibitor und einem Cathepsin-Inhibitor gewählt ist.

14. Verwendung nach Anspruch 9 bis 13, wobei die Nährstoffzusammensetzung eine Säuglingsanfangsnahrung, eine Folgenahrung oder eine Wachstumsmilch ist.

## Revendications

1. Composition nutritionnelle comprenant un produit fermenté dérivé du lait qui est fermenté par des bactéries productrices d'acide lactique, le produit fermenté dérivé du lait comprenant i) de l'acide lactique et/ou du lactate, ii) au moins un élément choisi dans le groupe constitué de lactosérum, de protéine lactosérique, et de caséine, destinée à être utilisée dans le traitement et/ou la prévention d'inflammations gastro-intestinales induites par une activité protéase de thiol en réduisant l'activité protéase de thiol, dans laquelle ledit produit dérivé du lait est fermenté avec du *Streptococcus thermophilus (S. thermophilus).*

2. Composition nutritionnelle destinée à être utilisée selon la revendication 1, dans laquelle la composition nutritionnelle présente une activité inhibitrice de la protéase de thiol d'au moins 40 unités inhibitrices de la papaïne (PIU) par g de poids sec.

3. Composition nutritionnelle destinée à être utilisée selon la revendication 1 ou 2, dans laquelle la composition nutritionnelle comprend entre 0,10 et 1,5 % en poids de la somme d'acide lactique et de lactate par rapport au poids sec de la composition nutritionnelle, dans laquelle la somme d'acide L-lactique et de L-lactate est supérieure à 50 % en poids de la somme totale d'acide lactique et de lactate.

4. Composition nutritionnelle destinée à être utilisée selon la revendication 1 à 3, dans laquelle les bactéries productrices d'acide lactique dans la composition nutritionnelle sont inactivées et/ou non-répliquantes.

5. Composition nutritionnelle destinée à être utilisée selon l'une quelconque des revendications 1 à 4, dans laquelle le produit dérivé du lait comprend de la caséine et des protéines lactosériques selon un rapport de poids de 20 : 80 à 80 : 20 et comprend au moins 8 % en poids de protéines par rapport au poids sec de la composition nutritionnelle.

6. Composition nutritionnelle destinée à être utilisée selon l'une quelconque des revendications 1 à 5, dans laquelle l'inhibiteur de protéase de thiol est choisi entre l'inhibiteur de la papaïne et l'inhibiteur de la cathepsine.

7. Composition nutritionnelle destinée à être utilisée selon l'une quelconque des revendications 1 à 6, dans laquelle la composition nutritionnelle est une préparation pour nourrisson, une préparation de suite ou un lait de croissance.

8. Composition nutritionnelle destinée à être utilisée selon l'une quelconque des revendications 1 à 7, dans laquelle ladite utilisation comprend l'administration de ladite composition nutritionnelle à un sujet humain âgé de 0 à 36 mois.

9. Utilisation de la fermentation par *S. thermophilus* d'une composition nutritionnelle comprenant du lait ou comprenant du lactose et au moins un élément choisi dans le groupe constitué de lactosérum, de protéine lactosérique et de caséine, pour augmenter l'activité de protéase de thiol d'une composition nutritionnelle.

10. Utilisation selon la revendication 9, dans laquelle la composition nutritionnelle comprend entre 0,10 et 1,5 % en poids de la somme d'acide lactique et de lactate par rapport au poids sec de la composition nutritionnelle, dans laquelle la somme d'acide L-lactique et de L-lactate est supérieure à 50 % en poids de la somme totale d'acide lactique et de lactate.

11. Utilisation selon la revendication 9 ou 10, dans laquelle les bactéries productrices d'acide lactique dans la composition nutritionnelle sont inactivées et/ou non-répliquantes.

12. Utilisation selon la revendication 9 à 11, dans laquelle la composition nutritionnelle comprend de la caséine et des protéines lactosériques selon un rapport de poids de 20 : 80 à 80 : 20 et comprend au moins 8 % en poids de protéines par rapport au poids sec de la composition nutritionnelle.

13. Utilisation selon la revendication 9 à 12, dans laquelle l'inhibiteur de protéase de thiol est choisi entre l'inhibiteur de la papaïne et l'inhibiteur de la cathepsine.

14. Utilisation selon la revendication 9 à 13, dans laquelle la composition nutritionnelle est une préparation pour nourrisson, une préparation de suite ou un lait de croissance.
